# EUROPÄISCHE PATENTSCHRIFT

(11) **EP 0 444 097 B1**
(45) Veröffentlichungstag und Bekanntmachung des Hinweises auf die Patenterteilung: **09.11.1994**
(21) Anmeldenummer: 89912986.0
(22) Anmeldetag: 21.11.1989
(51) Int. Cl.: A61N 1/04, A61H 39/08

(54) **PUNKTUAL-STIMULATIONS-NADEL UND PUNKTUAL-STIMULATIONS-THERAPIEGERÄT**
NEEDLE AND THERAPEUTIC DEVICE FOR STIMULATING SPECIFIC POINTS OF THE BODY
AIGUILLE ET DISPOSITIF THERAPEUTIQUE POUR STIMULER DES POINTS SPECIFIQUES DU CORPS

(30) Priorität: 21.11.1988 AT 2846/88; 21.11.1988 AT 2850/88
(43) Veröffentlichungstag der Anmeldung: 04.09.1991
(73) Patentinhaber: SZELES, Josef Constantin, A-1190 Wien (AT)
(72) Erfinder: SZELES, Josef Constantin, A-1190 Wien (AT)
(74) Vertreter: Weinzinger, Arnulf, Dipl.-Ing.
(86) Internationale Anmeldenummer: AT8900107
(87) Internationale Veröffentlichungsnummer: WO9005560

(56) Entgegenhaltungen:
- DE-A- 3 437 493
- FR-A- 969 374
- FR-A- 2 345 994
- FR-A- 2 392 650

## Beschreibung

Die Erfindung bezieht sich auf eine Punktual-Stimulations-Nadel gemäß Oberbegriff des Anspruchs 1.

Die Erfindung bezieht sich weiter auf ein Punktual-Stimulations-Therapiegerät mit einem tragbaren batteriebetriebenen Behandlungsstromgenerator und mit Elektroden, welche an diesen Behandlungsstromgenerator angeschlossen sind.

An einer Anzahl von Stellen des Körpers sind unter der Hautoberfläche Rezeptorenbereiche gelegen, die in reizleitender Verbindung mit an anderen Stellen des Körpers gelegenen Körperteilen stehen. Durch Stimulation solcher Rezeptorenbereiche kann zu diagnostischen oder therapeutischen Zwecken auf die mit diesen Rezeptorenbereichen in Verbindung stehenden Körperteile Einfluß ausgeübt werden.
Punktual-Stimulations-Nadeln dienen dazu, bei einem Einstich in Rezeptorenbereiche, welche an der Körperoberfläche bzw. in der Haut liegen und in reizleitender Verbindung mit bestimmten Körperteilen stehen, eine Stimulation dieser Rezeptorenbereiche herbeizuführen um auf die diesen Stellen zugeordneten Körperteile einzuwirken. Eine solche Einwirkung ist auch das Ziel der Akupunktur.

Die für die Akupunktur üblicherweise verwendeten Stimulationsnadeln haben einen Einstichteil, der eine glatte Oberfläche aufweist und in dem an die Spitze anschließenden Bereich einen Durchmesser von ungefähr 0.25 mm hat. Dieser Einstichteil hat meist eine Länge von etwa 10 bis 15 mm, und es schließt sich daran der Halteteil an, welcher meist annähernd zylindrisch ausgebildet ist und einen Durchmesser von etwa 2 bis 3 mm hat.

Es ist für die Feststellung von Zuordnungen zwischen Rezeptorenbereichen und Körperteilen und auch für das Stimulieren von Rezeptorenbereichen durch Einstechen von Punktual-Stimulations-Nadeln von wesentlicher Bedeutung, daß die Punktual-Stimulations-Nadeln möglichst genau an jenen Stellen eingestochen werden, an denen sich die Rezeptorenbereiche befinden. Es ist weiter wichtig, daß die Punktual-Stimulations-Nadeln, sobald sie an der richtigen Stelle eingestochen worden sind, dort einen guten Halt haben, damit die angestrebte Wirkung über einen längeren Zeitraum aufrecht bleibt. Beiden Erfordernissen wird mit den vorerwähnten üblicherweise bei der Akupunktur verwendeten Nadeln in der Regel nur mangelhaft entsprochen. Um den Nadeln mit glattem Einstichteil einen hinreichend stabilen Halt in der Haut zu geben, werden diese Nadeln in der Regel so tief eingestochen, daß ein solcher Sitz der Nadeln gewährleistet ist, wobei diese Einstichtiefe oft deutlich größer ist als die Tiefe in der die zu stimulierenden Rezeptorenbereiche liegen und ein solches verhältnismäßig tiefes Einstechen der Nadeln wird meist als unangenehm empfunden; die Stichempfindung ist der Stimulationswirkung überlagert und kann diese Wirkung schmälern. Es macht sich weiter beim Einstechen solcher Nadeln oft auch der Eindringwiderstand nachteilig bemerkbar, dahingehend, daß sich beim Einstechen Verschiebungen ergeben, die das exakte Erreichen der Rezeptorenbereiche beeinträchtigen.

Es kann erwähnt werden, daß aus der DE-A-28 23 307 Elektroden bekannt sind, welche zum Einstechen in die Haut vorgesehen sind und zur Erfassung elektrophysiologischer Spannungen (EKG) dienen sollen. Diese bekannten Elektroden bestehen aus einem zum Einstechen in die Haut vorgesehenen, an einem Ende zugespitzten Stift, der eine oder mehrere knollenartige Ausbauchungen besitzt, und aus einem annähernd zylindrischen Basisteil, in dem der Stift befestigt ist. Ein exakt gezieltes Einstechen dieser Elektroden unter die Haut wird durch die genannten Ausbauchungen beeinträchtigt. Es ist auch eine Ausführungsform mit einer schraubenartig gewundenen Ausbauchung dargestellt.

Es ist ein Ziel der vorliegenden Erfindung eine Punktual-Stimulations-Nadel zu schaffen, welche ein leichtes und feinfühlig steuerbares Eindringen des Einstichteiles in die Haut ermöglicht, und welche gleichzeitig auch bei geringer Einstichtiefe einen sicheren Sitz an der Einstichstelle erzielen läßt.

Die erfindungsgemäße Punktual-Stimulations-Nadel eingangs erwähnter Art ist dadurch gekennzeichnet, daß der Einstichteil ein ein- oder mehrfaches Gewinde trägt, dessen Gewindeganghöhe größer als das Zweifache des Durchmessers des Einstichteiles an seiner dicksten Stelle ist, daß das Schraubengewinde außen einen scharfen Gewindegrat aufweist, und daß der Halteteil in Form eines Ringes oder Plättchens ausgebildet ist. Durch diese Ausbildung kann der vorstehend angeführten Zielsetzung sehr gut entsprochen werden. Die schraubenförmige Ausbildung des Einstichteiles ermöglicht unter Anwendung einer leichten Drehbewegung ein feinfühliges Einführen der Nadelspitze in die Haut, wobei auch die Eindringtiefe gut gesteuert werden kann, und der Einstichvorgang durch den scharfen Gewindegrat leichtgängig und wenig gewebeverdrängend abläuft. Durch eine solche Ausbildung kann in Verbindung mit einer feinfühligen Handhabung auch das beim Einstechen oft empfundene Unbehagen auf ein Minimum reduziert werden. Es ist dabei gleichzeitig auch vorteilhaft, wenn das Schraubengewinde im Profilquerschnitt gesehen an den Gewindegrat anschließend eine flache Mulde aufweist, deren Krümmungsradius annähernd so groß wie der Radius der Nadel ist. Es wird weiter durch die Schraubenform des Einstichteiles der Nadel auch bei verhältnismäßig geringer Einstichtiefe ein guter Halt der Nadel erzielt. Die vorgesehene Ausbildung des Halteteiles der Nadel in Form eines Ringes oder Plättchens läßt beim Einführen und beim Entfernen der Nadel das Ausmaß der Drehung leicht erkennen und läßt überdies eine gute Abstützung der Nadel auf dem die Einstichstelle umgebenden Hautbezirk erzielen, was vorteilhaft zu einem sicheren Sitz der Nadel beiträgt. Durch die Ausbildung des Halteteiles kann man weiter auch die Nadel mit einem den Halteteil überdeckenden Heftpflaster zusätzlich fixieren, so daß über längere Zeiträume ein exakter Sitz der Nadel gewährleistet ist und gleichzeitig die Einstichstelle gegen Verschmutzung weitgehend geschützt ist.

Es hat sich in vielen Fällen eine Ausführungsform als günstig erwiesen, welche dadurch gekennzeichnet ist, daß die den Einstichteil bildende Schraube etwa 1/4 bis 1/2 Gang aufweist.

Die Länge des Einstichteiles der Nadel wird für die meisten Einsatzfälle vorteilhaft kleiner als 4 mm gewählt. Für die Stimulation von am Körper liegenden Rezeptorenbereichen wird vorteilhaft eine Länge des Einstichteiles der Nadel von etwa 3 mm gewählt. Für eine Aurikulo-Stimulation ist eine Länge des Einstichteiles von etwa 1 bis 2 mm günstig, wobei für Nadeln, die für einen Einstich in verknorpelte Bereiche des Ohres vorgesehen sind, vorteilhaft die Länge des Einstichteiles mit etwa 1 mm gewählt wird und für einen Einstich in andere Stellen des Ohres eine Länge des Einstichteiles von etwa 2 mm günstig ist.

Um die Stütz- und Haltewirkung des Halteteiles der Punktual-Stimulations-Nadel weiter zu verbessern kann man vorteilhaft auf der an der Haut zur Anlage kommenden Seite des Halteteiles einen Haftkleber vorsehen, der z.B. in Form eines Klebstoffauftrages oder in Form eines Ringes einer selbstklebenden Folie ausgebildet sein kann.

Man hat schon eine Stimulation von Rezeptorenbereichen durch Zufuhr elektrischer Ströme versucht, und hiezu in der Regel Elektroden verwendet, die auf der Hautoberfläche aufliegen. Diese Technik wird manchmal "Elektro-Akupunktur" genannt. Es ist ein auf diese Technik abgestimmtes Stimulations-Therapiegerät bekannt, welches einen sehr kleinen batteriebetriebenen Behandlungsstromgenerator aufweist, der durch seine geringe Größe leicht am Körper getragen werden kann, und der über flexible Leitungen mit einem Elektrodenträger verbunden ist, der mehrere Stiftelektroden trägt, die an ihrer Vorderseite abgerundet sind, und der an die zu behandelnde Stelle des Körpers aufgesetzt wird. Ein wesentlicher Nachteil dieses Gerätes und der mit auf die Hautoberfläche aufgelegten Elektroden arbeitenden Elektro-Akupunktur überhaupt, liegt darin, daß diese Art der Elektro-Akupunktur sehr oft nur eine geringe Wirkung zu erbringen vermag, bzw. daß bei einer solchen Behandlung überhaupt keine merkliche Wirkung erzielt wird.

Auch bei einer aus der FR-A-969 374 bekannten Vorrichtung, bei der an einem als Träger dienenden Handschuh mehrere Elektroden an den Spitzen der Handschuhfinger angebracht sind und ein mehradriges biegsames Kabel zur Verbindung dieser Elektroden mit einer Behandlungsstromquelle vorgesehen ist, erfolgt eine Kontaktierung der Hautoberfläche mit den Elektroden; es ist dabei unter anderem eine Elektrodenform vorgesehen, bei der in der Mitte der Stirnfläche eines zylinderähnlichen Körpers eine kegelige Spitze angeordnet ist.

Es konnte festgestellt werden, daß eine direkte punktuelle Stimulation der Rezeptorenbereiche nahezu in allen Fällen eine gute therapeutische Wirkung zu erbringen vermag. Die üblicherweise bei der Akupunktur zur Stimulation verwendeten langen Stimulationsnadeln können aber kaum über längere Zeit vom Patienten getragen werden, weil die Lage dieser Nadeln, d.h. ihr Halt und ihre Einstichtiefe durch äußere Kräfte in unerwünschter Weise verändert werden kann. Überdies nimmt häufig die Stimulationswirkung von solchen Nadeln nach einiger Zeit beträchtlich ab.

Es ist nun ein weiteres Ziel der vorliegenden Erfindung ein Punktual-Stimulations-Therapiegerät zu schaffen, mit dem auf praktisch gangbare Weise eine Stimulation von Rezeptorenbereichen über längere Zeiträume vorgenommen werden kann.

Die Erfindung schafft ein Punktual-Stimulations-Therapiegerät eingangs erwähnter Art, welches dadurch gekennzeichnet ist, daß mindestens eine Elektrode eine über eine flexible Leitung mit dem Behandlungsstromgenerator verbundene Punktual-Stimulations-Nadel ist, deren Einstichteil kürzer als 4 mm ist, und ein ein- oder mehrfaches Gewinde trägt, dessen Gewindeganghöhe größer als das Zweifache des Durchmessers des Einstichteiles der betreffenden Elektrode(n) an seiner dicksten Stelle ist, wobei das Schraubengewinde außen einen scharfen Gewindegrat aufweist, welche Stimulations-Nadel einen an den Einstichteil anschließenden Halteteil in Form eines Ringes oder Plättchens aufweist, und daß vorzugsweise am Gehäuse des Behandlungsstromgenerators eine Flächenelektrode vorgesehen ist. Durch diese Ausbildung kann der vorstehend angeführten Zielsetzung sehr gut entsprochen werden. Es kann dadurch, daß mindestens eine Elektrode des Gerätes eine über eine flexible Leitung mit dem Behandlungsstromgenerator verbundene Punktual-Stimulations-Nadel ist, ein eine Stimulation bewirkender elektrischer Strom unmittelbar in einen Rezeptorenbereich eingeleitet werden, wobei dadurch, daß der Behandlungsstromgenerator ohne weiteres am Körper getragen werden kann, die Stimulation auch über längere Zeiträume hindurch vorgenommen werden kann; die spezielle Ausbildung der Punktual-Stimulations-Nadel, die beim erfindungsgemäßen Punktual-Stimulations-Therapiegerät vorgesehen ist, ermöglicht eine einfache Handhabung beim Setzen der Nadel und läßt einen über längere Zeiträume sicheren Sitz der Nadel erzielen, der gegenüber mäßigen von außen einwirkenden Kräften bestandfähig ist, da der Halteteil der Nadel eine gute Abstützung an dem die Einstichstelle umgebenden Hautbereich erfährt, und da die Nadel ohne weiteres zusätzlich mit einem überdeckenden Heftpflaster geschützt werden kann.

Es ist dieses Gerät insbesondere für die Aurikular-Therapie, bei der die die Elektrode bildende Punktual-Stimulations-Nadel in Rezeptorenbereiche an der Ohrmuschel eingestochen wird, gut geeignet.

Es ermöglicht die vorgesehene Ausbildung der Nadelelektrode(n) zum einen ein besonders feinfühliges, leichtgängiges und exaktes Einstechen der Elektrode bzw. Elektroden, wodurch das Treffen der Rezeptorenbereiche erleichtert und das üblicherweise beim Einstechen von Nadeln in die Haut verspürte Unbehagen weitgehend gemildert werden kann, und es wird zum anderen durch diese Ausbildung des Einstichteils der Elektrode eine weitere Verbesserung des Haltes der Elektrode an der Einstichstelle erzielt.

Die Länge des Einstichteiles der die Elektrode(n) bildenden Punktual-Stimulations-Nadel(n) wird für die Stimulation von am Körper bzw- Rumpf oder an Extremitäten liegenden Rezeptorenbereichen vorteilhaft mit etwa 3 mm gewählt. Für die Aurikulotherapie ist eine Länge des Einstichteiles von etwa 1 bis 2 mm günstig, wobei für Elektroden, die für einen Einstich im verknorpelten Bereiche des Ohres vorgesehen sind, vorteilhaft die Länge des Einstichteiles mit etwa 1 mm gewählt wird und für einen Einstich in andere Stellen des Ohres eine Länge des Einstichteiles von etwa 2 mm günstig ist.

Um die Stütz- und Haltewirkung des Halteteiles der die Elektrode(n) bildenden Punktual-Stimulations-Nadel(n) weiter zu verbessern, kann man vorteilhaft auf der an der Haut zur Anlage kommenden Seite des Halteteiles einen Haftkleber vorsehen, der z.B. in Form eines Klebstoffauftrages oder in Form eines Ringes einer selbstklebenden Folie ausgebildet sein kann.

Es ist auch günstig eine Klebefixierung der flexiblen Zuleitung(en) zu der (den) Elektrode(n) an der Haut vorzunehmen. Auch hiezu kann an der bzw. den Zuleitung(en) ein Klebstoffauftrag vorgesehen werden oder an der bzw. den Zuleitung(en) eine dünne selbstklebende Folie vorgesehen werden.

Bildet man den Einstichteil der Elektrode bzw. der Elektroden als Schraube aus, ist es in vielen Fällen günstig, eine möglichst hohe Gewindeganghöhe vorzusehen, z.B. eine Gewindeganghöhe, welche über die Länge des Einstichteiles der Nadel etwa 1/4 bis 1/2 Gang ergibt.

Hinsichtlich der Nadel, und zwar sowohl hinsichtlich der Herstellung als auch hinsichtlich der Manipulation derselben, ist eine Ausführungsform des erfindungsgemäßen Punktual-Stimulations-Therapiegerätes sehr günstig, welche dadurch gekennzeichnet ist, daß der Halteteil ein aus Draht gebogener Ring ist, wobei ein Ende dieses Drahtes radial zur Ringmitte abgebogen ist und von dort ausgehend abermals abgebogen den Einstichteil bildet.

Durch die mit einer Punktual-Stimulations-Nadel erfolgende Einleitung der Stimulation in die Rezeptorenbereiche selbst, kann die Stimulation mit dem erfindungsgemäß ausgebildeten Punktual-Stimulations-Therapiegerät mit sehr geringen Stromstärken, in der Regel einige Mikroampere, das Auslangen finden; dadurch braucht der im Gerät vorzusehende Impulsgenerator nur eine verhältnismäßig geringe Leistung abzugeben und kann demgemäß über längere Zeiträume von einer verhältnismäßig kleinen Batterie gespeist werden. Man kann so insgesamt verhältnismäßig kleine Abmessungen des Behandlungsstromgenerators erzielen, so daß derselbe an beliebigen Körperstellen, z.B. unmittelbar hinter dem Ohr, getragen werden kann. Die bei einer bevorzugten Ausbildung des erfindungsgemäßen Punktual-Stimulations-Therapiegerätes am Gehäuse des Behandlungsstromgenerators vorgesehene Flächenelektrode kann mit einem Pol des Ausganges des Behandlungsstromgenerators in Verbindung stehen, so daß zur Stimulation nur mehr eine Nadelelektrode verwendet werden muß; es ist aber auch möglich gewünschtenfalls mehrere Elektroden in Form von Punktual-Stimulations-Nadeln, welche wie vorstehend angeführt ausgebildet sind, einzusetzen und beide Ausgangsklemmen des Behandlungsstromgenerators mit Akupunkturnadeln zu verbinden.

Das erfindungsgemäße Punktual-Stimulations-Therapiegerät kann im Hinblick auf seinen einfachen Aufbau auch als Einmalgerät ausgebildet werden, was aus hygienischen Gründen besonders vorteilhaft ist.

Zum Festhalten des Behandlungsstromgenerators am Körper, z.B. hinter dem Ohr, kann im einfachsten Fall ein Formschluß oder ein Heftpflaster vorgesehen sein. Es ist auch günstig an der Außenseite des Gehäuses des Behandlungsstromgenerators einen Haftkleber vorzusehen.

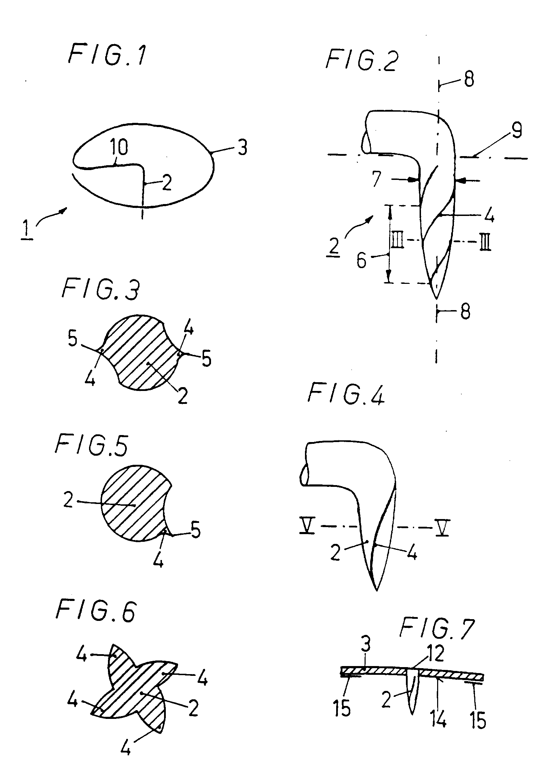

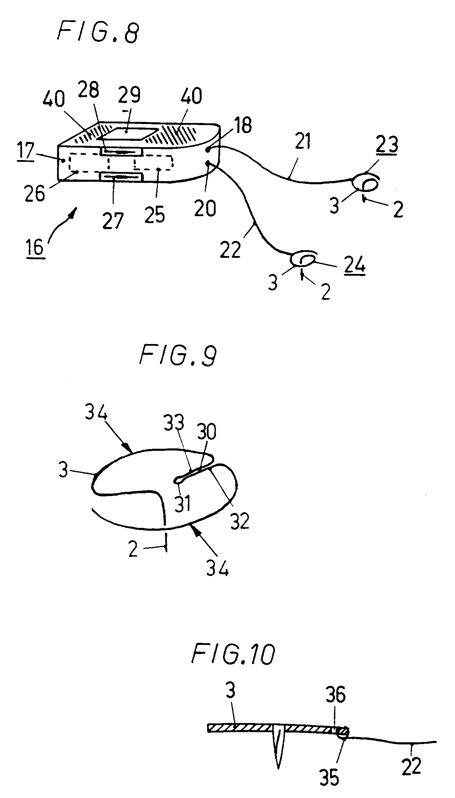

Die Erfindung wird nun unter Bezugnahme auf Beispiele, welche in der Zeichnung dargestellt sind, weiter erläutert. In der Zeichnung zeigt Fig. 1 eine erste Ausführungsform einer erfindungsgemäß ausgebildeten Punktual-Stimulations-Nadel in einer Ansicht, Fig. 2 gleichfalls in einer Ansicht den Einstichteil einer solchen Nadel in gegenüber Fig. 1 vergrößertem Maßstab, Fig. 3 in weiter vergrößertem Maßstab einen Schnitt nach der Linie III-III in Fig. 2, Fig. 4 eine Variante des Einstichteiles einer solchen Nadel in einer der Fig. 2 entsprechenden Darstellung, Fig. 5 einen Schnitt gemäß der Linie V-V in Fig. 4 in weiter vergrößertem Maßstab, Fig. 6 eine weitere Variante des Einstichteiles in einer den Fig. 3 und 5 entsprechenden Schnittdarstellung, und Fig. 7 eine weitere Ausführungsform einer erfindungsgemäß ausgebildeten Punktual-Stimulations-Nadel in einer Schnittdarstellung; Fig. 8 zeigt eine Ausführungsform eines erfindungsgemäßen Punktual-Stimulations-Therapiegerätes in einer Ansicht, Fig. 9 eine Elektrode mit integrierter Anschlußklemme und Fig. 10 eine über eine Anschlußklemme mit einer Leitung verbundene Elektrode.

Die in den Fig. 1 bis 3 dargestellte Ausführungsform einer Punktual-Stimulations-Nadel 1 weist einen Einstichteil 2 und einen Halteteil 3 auf. Der Einstichteil 2 ist als zweigängige Schraube ausgebildet, wobei das Schraubengewinde 4 außen scharfe Gewindegrate 5 aufweist. Die Ganghöhe 6 des Schraubengewindes 4 ist größer als das Zweifache des Durchmessers 7, den der Einstichteil an seiner dicksten Stelle hat. Das Schraubengewinde 4 hat, wie aus Fig. 3 unmittelbar ersichtlich ist, ein seichtes Profil.

Der Halteteil 3 ist in Form eines Ringes ausgebildet, dessen dem Einstichteil zugewandte Seite in einer zur geometrischen Achse 8 des Einstichteiles 2 annähernd senkrecht verlaufenden Ebene 9 liegt. Es ist dabei der Halteteil 3 aus Draht gebogen, wobei ein Ende 10 dieses Drahtes radial zur Ringmitte hin abgebogen ist und von dort ausgehend, abermals abgebogen, den Einstichteil 2 bildet.

Die Länge des Einstichteiles 2 beträgt bei dieser Ausführungsform ca. 2 mm, und es ist der Durchmesser des Ringes 3 annähernd 4 mm. Die Dicke 7 des Einstichteiles beträgt ca. 0,3 mm.

Bei der in den Fig. 4 und 5 dargestellten Variante ist der Einstichteil 2 in Form einer eingängigen Schraube ausgebildet, wobei das Schraubengewinde 4 wieder, wie Fig. 5 zeigt, außen einen scharfen Gewindegrat 5 aufweist und ein seichtes Profil hat. Die den Einstichteil 2 bildende Schraube hat in diesem Fall 1/2 Gang. Die Länge des Einstichteiles beträgt in diesem Fall ca. 1 mm.

Bei der in Fig. 6 dargestellten Variante ist der Einstichteil 2 als viergängige Schraube ausgebildet, wobei das Schraubengewinde 4 eine größere Profiltiefe als bei den in den Fig. 3 und 5 dargestellten Ausführungsformen hat.

Bei der Ausführungsform nach Fig. 7 ist der Halteteil 3 in Form eines Plättchens ausgebildet, welches in seiner Mitte 12 mit dem Einstichteil 2 verbunden ist. Der Einstichteil 2 kann dabei in eine entsprechende Öffnung im Plättchen form- und/oder kraftschlüssig eingefügt sein oder z.B. durch eine Löt- oder Schweißverbindung mit dem Plättchen verbunden sein. Man kann auch eine formschlüssige Verbindung zusammen mit einer Löt- oder Schweißverbindung vorsehen. Die Ausbildung des Einstichteiles kann auch in diesem Fall in verschiedener Weise vorgenommen werden, z.B. wie dies in den Fig. 2 bis 6 dargestellt ist.

An der an der Haut zur Anlage kommenden Seite 14 des Halteteiles 3 kann ein Haftkleber 15, z.B. in Form eines Klebstoffauftrages oder in Form eines Ringes einer selbstklebenden Folie, angeordnet sein.

Das in Fig. 8 schematisch dargestellte Punktual-Stimulations-Therapiegerät 16 weist einen Behandlungsstromgenerator 17 auf, an dessen Ausgangsklemmen 18, 20 über flexible Leitungen 21, 22 Elektroden 23, 24 angeschlossen sind. Im Gehäuse des Behandlungsstromgenerators 17 ist ein elektrischer Impulsgeber bzw. Funktionsgenerator 25 angeordnet, der die Behandlungsströme erzeugt und gewünschtenfalls hinsichtlich Impulsform bzw. Wellenform, Impulsfrequenz bzw. Wellenfrequenz und Impulsamplitude bzw. Wellenamplitude sowie gegebenenfalls hinsichtlich von Impulsabständen umschaltbar ausgebildet ist. Dieser Impulsgeber bzw. Funktionsgenerator wird von einer in das Gehäuse des Behandlungsstromgenerators 17 eingesetzten Batterie 26 gespeist. Ein Schalter 27 ist zum Ein- und Ausschalten des Impulsgebers vorgesehen. Ein weiterer Schalter 28 dient zum Anschalten einer am Gehäuse des Behandlungsstromgenerators 17 vorgesehenen Flächenelektrode 29 an eine der Ausgangsklemmen 18, 20 des Behandlungsstromgenerators 17.

Die Elektroden 23, 24 sind Punktual-Stimulations-Nadeln, deren Einstichteil 2 kürzer als 4 mm ist, und die einen an den Einstichteil 2 anschließenden Halteteil 3, der in Form eines Ringes oder Plättchens ausgebildet ist, aufweisen. In vielen praktischen Fällen, z.B. für die Aurikulo-Therapie, kann eine Länge des Einstichteiles von etwa 1 mm bis 2 mm vorgesehen werden. Die dem Einstichteil 2 zugewandte Seite des als Ring oder Plättchen ausgebildeten Halteteils 3 liegt in einer zur geometrischen Achse 8 des Einstichteils 2 annähernd senkrecht verlaufenden Ebene, welche in Fig. 2 durch eine strichpunktierte Linie 9 angedeutet ist. Es wird durch diese Ausbildung des Halteteiles der eine Elektrode bildenden Punktual-Stimulations-Nadel eine gute Abstützung einer in eine Hautstelle eingestochenen Nadel auf dem die Einstichstelle umgebenden Hautbezirk erzielt, was vorteilhaft zu einem sicheren Sitz der Nadel beiträgt. Man kann durch diese Ausbildung des Halteteiles die Nadel auch weiter mit einem den Halteteil überdeckenden Heftpflaster zusätzlich fixieren, so daß über längere Zeiträume ein exakter Sitz der Nadel gewährleistet ist und gleichzeitig die Einstichstelle gegen Verschmutzung weitgehend geschützt ist.

Bei der in Fig. 8 dargestellten Ausführungsform ist wie bei der Nadel nach Fig. 1 bis 4 der Halteteil 3 ein aus Draht gebogener Ring, wobei ein Ende des Drahtes radial zur Ringmitte abgebogen ist und von dort ausgehend abermals abgebogen den Einstichteil 2 bildet.

Die Elektroden 23, 24 können vorteilhaft in Form der in den Fig. 1 bis 7 dargestellten Nadeln ausgebildet sein.

Die in Form einer speziellen Punktual-Stimulations-Nadel ausgebildeten Elektroden können zur Bildung einer elektrischen Verbindung mit der vom Behandlungsstromgenerator kommenden flexiblen Leitung 21, 22 im einfachsten Fall verlötet oder verschweißt sein, oder es kann eine solche Leitung mit der betreffenden Elektrode durch eine Klemmverbindung verbunden sein. Eine Klemmverbindung kann dabei gewünschtenfalls auch lösbar ausgebildet sein, so daß man gewünschtenfalls verschiedene Elektroden, die sich z.B. hinsichtlich der Länge des Einstichteiles unterscheiden, einsetzen kann. Fig. 9 zeigt eine Ausführungsform einer Elektrode, welche ähnlich wie die in Fig. 1 dargestellte Elektrode bzw. Nadel ausgebildet ist, und eine in den Halteteil integrierte Verbindungsklemme 30 aufweist. Zur Bildung dieser Verbindungsklemme 30 ist der den Halteteil 3 bildende Ring mit einer U-förmigen Einbuchtung 31 versehen, deren Schenkel 32, 33 durch Zusammendrücken des Ringes im Sinne der Pfeile 34 aufgespreizt werden können; es kann solcherart die Klemme 30 geöffnet und das Ende einer elektrischen Leitung zwischen die Schenkel 32, 33 eingeführt werden, welche dann nach Wegfall der die Klemme öffnenden Kraft wieder zurückfedern und dabei die elektrische Leitung festhalten.

Auch an einer zu einer Elektrode führenden flexiblen Leitung 21, 22 kann man eine Verbindungsklemme 35 vorsehen und mit dieser Verbindungsklemme eine elektrische Verbindung der betreffenden Leitung zu der als Punktual-Stimulations-Nadel ausgebildeten Elektrode schaffen. Eine solche Ausführungsform ist in Fig. 10 dargestellt. Die an einer flexiblen Leitung 22 angebrachte Verbindungsklemme 35, welche in Form eines elastischen Ringes ausgebildet ist, greift mit ihrer einen Seite über den Rand eines in diesem Fall als Plättchen ausgebildeten Halteteils 3 der betreffenden Elektrode, und mit ihrem anderen Schenkel durch eine in diesem Halteteil 3 vorgesehene Öffnung 36 und es wird auf diese Weise ein sehr sicherer Halt der flexiblen Leitung 22 am Halteteil 3 der Elektrode erzielt.

Man kann zur Stimulation mit dem Punktual-Stimulations-Therapiegerät einen über zwei Elektroden, die in vorstehend erläuterter Art in Form spezieller Punktual-Stimulations-Nadeln ausgebildet sind, geleiteten Strom zur Anwendung bringen, wobei, wie dies in Fig. 1 dargestellt ist, die beiden Elektroden an die verschiedene Polarität aufweisenden Ausgangsklemmen des Behandlungsstromgenerators 17 angeschlossen sind. Gewünschtenfalls kann man auch bei dieser Art der Stimulation eine größere Anzahl von Elektroden einsetzen, welche über flexible Leitungen mit dem Behandlungsstromgenerator 17 verbunden sind. Eine solche Leitung kann dabei gegebenenfalls auch mehrere nacheinander aufgereihte Elektroden tragen. In vielen Fällen ist es günstig eine Flächenelektrode 29 an einen Pol des Stimulationsstromkreises der vom Behandlungsstromgenerator gespeist wird, anzuschalten und eine oder mehrere als Punktual-Stimulations-Nadel(n) ausgebildete Elektrode(n) an den anderen Pol dieses Stromkreises anzuschließen. Man kann aber auch gewünschtenfalls Punktual-Stimulations-Nadelelektroden an beide Ausgangsklemmen des Behandlungsstromgenerators anschließen und ergänzend an eine dieser Ausgangsklemmen eine Flächenelektrode anschließen; hiebei kann der Schalter 28 so ausgebildet sein, daß mit ihm die Flächenelektrode 29 wahlweise mit einer der beiden Ausgangsklemmen des Behandlungsstromgenerators 17 verbunden werden kann.

Neben der Flächenelektrode kann ein Haftkleber 40 zum Festhalten des Behandlungsstromgenerators am Körper vorgesehen sein.

## Patentansprüche

1. Punktual-Stimulations-Nadel mit einem Einstichteil (2) in Form eines geraden, sich zu einer Spitze gleichförmig verjüngenden Stiftes und mit einem Halteteil (3), dessen dem Einstichteil zugewandte Seite in einer zur geometrischen Achse (8) des Einstichteiles annähernd senkrecht verlaufenden Ebene (9) liegt, dadurch gekennzeichnet, daß der Einstichteil (2) ein ein- oder mehrfaches Gewinde trägt, dessen Gewindeganghöhe (6) größer als das Zweifache des Durchmessers (7) des Einstichteiles (2) an seiner dicksten Stelle ist, daß das Schraubengewinde (4) außen einen scharfen Gewindegrat (5) aufweist, und daß der Halteteil (3) in Form eines Ringes oder Plättchens ausgebildet ist.

2. Punktual-Stimulations-Nadel nach Anspruch 1, dadurch gekennzeichnet, daß die den Einstichteil (2) bildende Schraube etwa 1/4 bis 1/2 Gang aufweist.

3. Punktual-Stimulations-Nadel nach Anspruch 1 oder 2, dadurch gekennzeichnet, daß das Schraubengewinde (4) im Profilquerschnitt gesehen an den Gewindegrat anschließend eine flache Mulde aufweist, deren Krümmungsradius annähernd so groß wie der Radius der Nadel ist.

4. Punktual-Stimulations-Nadel nach einem der Ansprüche 1 bis 3, dadurch gekennzeichnet, daß die Länge des Einstichteiles (2) kleiner als 4 mm ist.

5. Punktual-Stimulations-Nadel nach Anspruch 4, dadurch gekennzeichnet, daß die Länge des Einstichteiles (2) etwa 1 bis 2 mm ist.

6. Punktual-Stimulations-Nadel nach einem der Ansprüche 1 bis 5, dadurch gekennzeichnet, daß der Halteteil (3) der Nadel auf der an der Haut zur Anlage kommenden Seite (14) einen Haftkleber (15) trägt.

7. Punktual-Stimulations-Therapiegerät mit einem tragbaren batteriebetriebenen Behandlungsstromgenerator (17) und mit Elektroden (23, 24), welche an diesen Behandlungsstromgenerator angeschlossen sind, dadurch gekennzeichnet, daß mindestens eine Elektrode eine über eine flexible Leitung mit dem Behandlungsstromgenerator verbundene Punktual-Stimulations-Nadel ist, deren Einstichteil (2) kürzer als 4 mm ist, und ein ein- oder mehrfaches Gewinde trägt, dessen Gewindeganghöhe (6) größer als das Zweifache des Durchmessers (7) des Einstichteiles (2) der betreffenden Elektrode(n) an seiner dicksten Stelle ist, wobei das Schraubengewinde (4) außen einen scharfen Gewindegrat (5) aufweist, welche Stimulations-Nadel einen an den Einstichteil (2) anschließenden Halteteil (3) in Form eines Ringes oder Plättchens aufweist, dessen dem Einstichteil zugewandte Seite in einer zur geometrischen Achse (8) des Einstichteiles (2) annähernd senkrecht verlaufenden Ebene (9) liegt, und daß vorzugsweise am Gehäuse des Behandlungsstromgenerators (17) eine Flächenelektrode (29) vorgesehen ist.

8. Punktual-Stimulations-Therapiegerät nach Anspruch 7, dadurch gekennzeichnet, daß die den Einstichteil (2) bildende Schraube etwa 1/4 bis 1/2 Gang aufweist.

9. Punktual-Stimulations-Therapiegerät nach einem der Ansprüche 7 oder 8, dadurch gekennzeichnet, daß der Halteteil (3) ein aus Draht gebogener Ring ist, wobei ein Ende (10) dieses Drahtes radial zur Ringmitte abgebogen ist und von dort ausgehend abermals abgebogen den Einstichteil (2) bildet.

10. Punktual-Stimulations-Therapiegerät nach einem der Ansprüche 7 bis 9, dadurch gekennzeichnet, daß der Halteteil (3) der Elektrode(n) auf der an der Haut zur Anlage kommenden Seite (14) einen Haftkleber (15) trägt.

11. Punktual-Stimulations-Therapiegerät nach einem der Ansprüche 7 bis 10, dadurch gekennzeichnet, daß an der bzw. den Zuleitung(en) (21, 22) zu der (den) Elektrode(n) (23, 24) ein Klebstoffauftrag oder eine selbstklebende Folie vorgesehen ist.

12. Punktual-Stimulations-Therapiegerät nach einem der Ansprüche 7 bis 11, dadurch gekennzeichnet, daß am Gehäuse des Behandlungsstromgenerators (17) ein Haftkleber (40) vorgesehen ist.

13. Punktual-Stimulations-Therapiegerät nach einem der Ansprüche 7 bis 12, dadurch gekennzeichnet, daß der Einstichteil (2) der Elektrode(n) kürzer als 2 mm, insbesondere etwa 1 mm lang, ist.

## Claims

1. A punctual stimulation needle comprising a puncture part (2) in the form of a straight pin uniformly tapering towards a tip, and a holding part (3) whose side facing the puncture part lies in a plane (9) extending approximately perpendicular to the geometrical axis (8) of the puncture part, characterized in that the puncture part (2) has a single or multiple thread whose pitch (6) is larger than twice the diameter (7) of the puncture part (2) at its thickest point, in that the screw thread (4) externally has a sharp thread ridge (5) and in that the holding part (3) is designed in the form of a ring or small plate.

2. A punctual stimulation needle according to claim 1, characterized in that the screw forming the puncture part (2) has a 1/4 to 1/2 thread.

3. A punctual stimulation needle according to claim 1 or 2, characterized in that the screw thread (4), viewed in the profiled cross section, has a flat shallow following upon the thread ridge, the radius of curvature of which shallow approximately equals the radius of the needle.

4. A punctual stimulation needle according to any one of claims 1 to 3, characterized in that the length of the puncture part (2) is smaller than 4 mm.

5. A punctual stimulation needle according to claim 4, characterized in that the length of the puncture part (2) is approximately 1 to 2 mm.

6. A punctual stimulation needle according to any one of claims 1 to 5, characterized in that the holding part (3) of the needle carries an adhesive (15) on its side (14) coming to abut on the skin.

7. A punctual stimulation therapy apparatus comprising a portable battery-powered treatment current generator (17) and electrodes (23, 24) connected to said treatment current generator, characterized in that at least one electrode is a punctual stimulation needle connected with the treatment current generator via a flexible line and whose puncture part (2) is shorter than 4 mm, carrying a single or multiple thread whose pitch (6) is larger than twice the diameter (7) of the puncture part (2) of the respective electrode(s) at its thickest point, the screw thread (4) externally having a sharp thread ridge (5), which stimulation needle comprises a holding part (3) in the form of a ring or small plate following upon the puncture part (2) and whose side facing the puncture part lies in a plane (9) extending approximately perpendicular to the geometrical axis (8) of the puncture part (2), and in that, preferably, a surface electrode (29) is provided on the housing of the treatment current generator (17).

8. A punctual stimulation therapy apparatus according to claim 7, characterized in that the screw forming the puncture part (2) has a 1/4 to 1/2 thread.

9. A punctual stimulation therapy apparatus according to any one of claims 7 or 8, characterized in that the holding part (3) is a ring bent of wire, one end (10) of said wire being bent off radially towards the ring centre and, departing therefrom, again being bent off to form said puncture part (2).

10. A punctual stimulation therapy apparatus according to any one of claims 7 to 9, characterized in that the holding part (3) of the electrode(s) carries an adhesive (15) on its side (14) coming to abut on the skin.

11. A punctual stimulation therapy apparatus according to any one of claims 7 to 10, characterized in that an adhesive layer or a self-adhesive film is provided on the feed line(s) (21, 22) to the electrode(s) (23, 24).

12. A punctual stimulation therapy apparatus according to any one of claims 7 to 11, characterized in that an adhesive (40) is provided on the housing of the treatment current generator (17).

13. A punctual stimulation therapy apparatus according to any one of claims 7 to 12, characterized in that the puncture part (2) of the electrode(s) is shorter than 2 mm, in particular, has a length of approximately 1 mm.

## Revendications

1. Aiguille de stimulation ponctuelle comportant un élément de ponction (2) en forme d'une tige droite diminuant uniformément en une pointe ainsi qu'un élément de support (3) dont la face en regard de l'élément de ponction se situe dans un plan (9) s'étendant à peu près perpendiculairement à l'axe géometrique (8) de l'élément de ponction, caractérisée en ce que l'élément de ponction (2) porte un filetage simple ou multiple dont le pas (6) est supérieur à deux fois le diamètre (7) de l'élément de ponction (2), sur l'endroit le plus épais de ce-ci, en ce que le filetage (4) a une arête de filet aigue (5) à l'extérieur, et en ce que l'élément de support (3) est conformé en tant qu'anneau ou flan.

2. Aiguille de stimulation ponctuelle selon la revendication 1, caractérisée en ce que la vis formant l'élément de ponction a un filet de 1/4 à 1/2 d'environ.

3. Aiguille de stimulation ponctuelle selon la revendication 1 ou 2, caractérisée en ce que le filetage (4), vu en section transversale de profile, comprend une dépression peu profonde suivant l'ârète de filet et dont le rayon de courbure est d'environ aussi grand que le rayon de l'aiguille.

4. Aiguille de stimulation ponctuelle selon les revendications 1 à 3, caractérisée en ce que la longueur de l'élément de ponction (2) est inférieure à 4 mm.

5. Aiguille de stimulation ponctuelle selon la revendication 4, caractérisée en ce que la longueur de l'élément de ponction (2) est 1 à 2 mm d'environ.

6. Aiguille de stimulation ponctuelle selon l'une des revendications 1 à 5, caractérisée en ce que l'élément de support (3) de l'aiguille, du côté (14) allant s'appuyer sur le peau, porte un auto-adhésif (15).

7. Appareil de thérapie de stimulation ponctuelle comportant un générateur de courant de traitement portable à piles et des électrodes (23, 24) reliées audit générateur de courant de traitement, caractérisé en ce qu'au moins une électrode est constituée d'une aiguille de stimulation ponctuelle connectée au générateur de courant de traitement par un conduit flexible et dont l'élément de ponction (2) est inférieure à 4 mm, et porte un filetage simple ou multiple dont le pas (6) est supérieur à deux fois le diamètre (7) de l'élément de ponction (2), de l'électrode ou des électrodes respective(s), sur l'endroit le plus épais de ce-ci, cependant que le filetage (4) a une arête de filet aigue (5) à l'extérieur, laquelle aiguille de stimulation ponctuelle comporte un élément de support (3) en forme d'anneau ou de flan suivant l'élément de ponction (2) et dont la face en regard de l'élément de ponction se situe dans un plan (9) s'étendant à peu près perpendiculairement à l'axe géometrique (8) de l'élément de ponction (2), et en ce qu'une électrode de surface (29) de préférence est prévue sur l'enceinte du générateur de courant de traitement (17).

8. Appareil de thérapie de stimulation ponctuelle selon la revendication 7, caractérisé en ce que la vis formant l'élément de ponction a un filet de 1/4 à 1/2 d'environ.

9. Appareil de thérapie de stimulation ponctuelle selon l'une des revendications 7 ou 8, caractérisé en ce que l'élément de support (3) est un anneau plié à partir d'un fil métallique, une extrémité (10) dudit fil étant détournée radialement vers le centre d'anneau et, partant de là, étant détournée de nouveau pour former l'élément de ponction (2).

10. Appareil de thérapie de stimulation ponctuelle selon l'une des revendications 7 à 9, caractérisé en ce que l'élément de support (3) de l'électrode ou des électrodes, du côté (14) allant s'appuyer sur le peau, porte un auto-adhésif (15).

11. Appareil de thérapie de stimulation ponctuelle selon l'une des revendications 7 à 10, caractérisé en ce qu'un enduit d'adhésif ou un film auto-adhésif est prévu sur le(s) conduit(s) d'alimentation (21, 22) allant respectivement à l'électrode ou aux électrodes (23, 24).

12. Appareil de thérapie de stimulation ponctuelle selon l'une des revendications 7 à 11, caractérisé en ce qu'un auto-adhésif (40) est prévu sur l'enceinte du générateur de courant de traitement (17).

13. Appareil de thérapie de stimulation ponctuelle selon l'une des revendications 7 à 12, caractérisé en ce la longueur de l'élément de ponction (2) de l'électrode ou des électrodes est inférieure à 2 mm, en particulier est 1 mm d'environ.
